# EUROPEAN PATENT SPECIFICATION

(11) **EP 4 017 379 B1**
(45) Date of publication and mention of the grant of the patent: **29.10.2025**
(21) Application number: 20765417.9
(22) Date of filing: 20.08.2020
(51) Int. Cl.: A61B 17/04, A61F 2/24

(54) **ANCHOR WITH ADAPTABLE LENGTH**
ANKERMIT ANPASSBARER LÄNGE
ANCRAGE À LONGUEUR ADAPTABLE

(30) Priority: 21.08.2019 US 201962889744 P
(43) Date of publication of application: 29.06.2022
(73) Proprietor: Boston Scientific Scimed Inc., Maple Grove, Minnesota 55311 (US)
(72) Inventor: KRUMPELMANN, Graham, Stillwater, Minnesota 55082 (US)
(74) Representative: Peterreins Schley
(86) International application number: PCT/US2020/047194
(87) International publication number: WO 2021/035050

(56) References cited:
- US-A1- 2018 228 610
- US-A1- 2018 263 777

## Description

### FIELD

The technology generally relates to implantable coronary medical devices and more particularly to a customizable coronary implant.

### BACKGROUND

Mitral insufficiency (MI) (also referred to as mitral regurgitation or mitral incompetence) is a form of heart disease where the mitral annulus dilates excessively and the valve leaflets no longer effectively close, or coapt, during systolic contraction. Regurgitation of blood occurs during ventricular contraction and cardiac output may decrease as a result. Surgical and endoluminal annuloplasty techniques have been introduced that aim to restore a mitral valve to its native configuration, for example by implanting an annuloplasty ring around a valve annulus. Variabilities in the anatomy of the heart may cause challenges when attempting to place the implant. It would be desirable to identify implants that may be customized to the variable anatomy of the heart.

US 2018/026377 A1 discloses annuloplasty devices including a plurality of helical tissue anchors. US 2018/0228610 A1 discloses heart valve implants including re-sizable frames.

### SUMMARY

The present invention is directed to a coronary implant as set forth in the appended claims. The following disclosure describes non-limiting examples of some embodiments. The embodiments disclosed herein each have several aspects no single one of which is solely responsible for the disclosure's desirable attributes. For instance, other embodiments of the disclosed systems and methods may or may not include the features described herein. Moreover, disclosed advantages and benefits can apply only to certain embodiments and should not be used to limit the disclosure.

In accordance with the invention, the implant includes a frame having a proximal end and a distal end, an anchor and an anchor housing coupled to one of the proximal end or distal end of the frame. The anchor housing includes an anchor sleeve disposed within a bore of the anchor housing, the anchor sleeve having a lumen extending therethrough including at least one feature disposed on an internal wall of the lumen for translatably engaging the anchor. The anchor sleeve translatable within the bore of the anchor housing and configured to limit translation of the anchor through the anchor housing. The anchor sleeve includes a collar disposed on a proximal end of the anchor sleeve to limit the translation of the anchor sleeve within the bore or the translation of the anchor within the anchor sleeve, or both. The collar includes a proximal surface and at least one driver engagement feature disposed on the proximal surface.

The anchor may further include an anchor head disposed at a proximal end, the anchor head including a drive coupler. The anchor head may include a distal surface having a sleeve engagement feature disposed thereon, the sleeve engagement feature configured to cooperate with a driver engagement feature of a drive tube to translate the anchor sleeve within the bore of the anchor housing.

In one embodiment, the anchor housing may be one of a plurality of anchor housings, the anchor sleeve may be one of a plurality of anchor sleeves, the anchor may be one of a plurality of anchors, and the plurality of anchor housings may be disposed about the frame. Each anchor housing may engage one of the plurality of anchor sleeves which supports one of the plurality of anchors, and each of the plurality of anchors may be coupled to one drive tube of a plurality of drive tubes for independent translation of the coupled anchor and an associated anchor sleeve. According to another aspect, a system for annuloplasty includes a delivery catheter having a proximal handle, a distal end and a plurality of drive tubes extending therethrough. The system further includes an implant, coupled to the plurality of drive tubes of the delivery catheter, where the implant may include a frame having a proximal end and a distal end. In one embodiment, the system may include a plurality of anchors, each anchor coupled to a drive tube of the plurality of drive tubes for independent translation of the anchor. A plurality of anchor housings may be coupled to one of the proximal end or distal end of the frame, each anchor housing including an anchor sleeve disposed within a bore of the anchor housing, the anchor sleeve having a lumen extending therethrough including at least one feature disposed on an internal wall of the lumen for translatably engaging at least one anchor. Each anchor sleeve may be translatable within the bore of the anchor housing to control a distal extent of travel of the at least one anchor through the at least one anchor housing.

The anchor sleeve includes a collar disposed on a proximal end of the anchor sleeve to limit one or both of the translation of the anchor sleeve within the bore and the translation of the anchor within the anchor sleeve. The collar includes a proximal surface and at least one driver engagement feature disposed on the proximal surface. Each anchor may further include an anchor head disposed at a proximal end, the anchor head including a drive coupler. At least one drive tube may include a distal driver configured to releasably couple to the anchor head to drive the anchor through the lumen of the anchor sleeve. In various embodiments, the distal driver may include a sleeve engagement feature extending radially from its distal end, the sleeve engagement feature configured to cooperate with the driver engagement feature on the proximal surface of the collar to translate the anchor sleeve within the bore of the anchor housing. The anchor head may include a distal anchor head diameter that is larger in size than a lumen diameter of the lumen of the anchor sleeve to preclude distal translation of the anchor head into the lumen of the anchor sleeve. The collar may extend radially from the lumen of the anchor sleeve and a collar diameter of the collar may be larger than a bore diameter of the bore of the anchor housing to preclude distal translation of the collar into the bore. The anchor sleeve may include at least one external engagement feature disposed on an external surface of the anchor sleeve, the bore may include at least one internal engagement feature disposed on a wall of the bore, and the at least one external engagement feature of the anchor sleeve may cooperate with the at least one internal engagement feature of the bore to translate the anchor sleeve within the bore of the anchor housing. The at least one feature disposed on an internal wall of the lumen of the anchor sleeve may be configured to cooperate with at least one edge of an anchor to translate the anchor within the anchor sleeve.

In one embodiment, the frame may include an expandable frame including at least two struts joined at distal ends to form a distal apex, at least two struts joined at proximal ends to form a proximal apex, and at least one anchor housing may be disposed about the distal apex of the frame. An actuator may be translatably disposed about the proximal apex of the frame to compress the frame. In various embodiments, each drive tube of the plurality of drive tubes may be independently controlled to customize translation of the anchor, translation of the anchor sleeve, or both.

Also a method of annuloplasty is described, wherein the annuloplasty system includes a frame having a proximal end and a distal end and a plurality of anchor housings coupled to one of the proximal end or distal end of the frame. Each anchor housing may include an anchor sleeve disposed within a bore of the anchor housing, each anchor including a proximal collar having a drive tube engagement feature. A plurality of anchors, each anchor including a proximal anchor head coupled to a drive tube and a distal tip, may be provided, wherein each anchor may be disposed within one of the anchor sleeves of the plurality of anchor housings. The method includes the steps of advancing each anchor through each anchor sleeve until translation of the proximal anchor head is precluded by the proximal collar of the anchor sleeve and the drive tube engagement feature engages a sleeve engagement feature of the drive tube, and independently advancing each anchor sleeve through the anchor housing by action of the drive tube engagement feature on the sleeve engagement feature to advance the distal tip of the anchor past the anchor housing into tissue to a customized extent until translation of the anchor sleeve is precluded. In one embodiment, the method may further include the steps of releasing the drive tube from the anchor sleeve while maintaining a coupling with the anchor when the translation of anchor sleeve is precluded and continuing to drive the anchor to pull tissue into the anchor.

With such an arrangement, the anchor sleeve may be used to limit the depth to which each individual anchor is driven to customize implant deployment in accordance with the variabilities of patient anatomy.

### BRIEF DESCRIPTION OF THE DRAWINGS

The foregoing and other features of the present disclosure will become more fully apparent from the following description and appended claims, taken in conjunction with the accompanying drawings. In the drawings, similar symbols typically identify similar components, unless context dictates otherwise. The illustrative embodiments described in the detailed description, drawings, and claims are not meant to be limiting. Other embodiments may be utilized, and other changes may be made. It will be readily understood that the aspects of the present disclosure, as generally described herein, and illustrated in the drawing, can be arranged, substituted, combined, and designed in a wide variety of different configurations, all of which are explicitly contemplated and made part of this disclosure.
FIG. 1 is a diagram of one embodiment of an implant comprising anchor housings as disclosed herein;
FIG. 2 is a diagram of anchoring components according to an embodiment disclosed herein;
FIGS. 3A and 3B illustrate representative components of one embodiment of a deployment system as disclosed herein;
FIGS. 4A - 4C provide perspective views of one embodiment of cooperating anchor drive tube/ collar features that may be used as described herein;
FIGS. 5A and 5B illustrate deployment of an implant as disclosed herein to a heart chamber;
FIGS. 6A-6C are diagrams illustrating an example of an implant anchoring process using one embodiment of an anchor housing assembly disclosed herein; and
FIG. 7 illustrates one embodiment of an implant with customized anchor depth comprising anchor housings as described herein.

### DETAILED DESCRIPTION

The present invention is directed to a coronary implant as set forth in the appended claims. Heart disease impedes patient cardiac output, which reduces patient quality of life and lifespan. When heart valves fail to property coapt, or close, during their cardiac cycle, blood may leak backwards into the atrium each time the left ventricle contracts. Various procedures have been implemented to overcome valve insufficiency. These procedures include methods for restoring structural integrity to the valve annulus (a fibrous ring partially surrounding a heart valve) and/or repairing flailing leaflets resulting from stretched or torn chordae. Many of these methods involve anchoring an implant or other object to heart tissue. For example, annuloplasty may involve securing a ring or frame around a valve annulus using anchors.

One problem with such solutions arises because the anatomy of a heart valve, including the thickness of tissue surrounding the heart valve, varies along its circumference. Uniform deployment of anchors to the same depth may cause anchors to pierce too deeply into cardiac tissue, potentially reducing the effectiveness of the procedure.

According to one aspect, these problems are overcome through the use of an implant having an improved anchor housing assembly. In embodiements of the invention, the anchor housings are coupled to one of the proximal end or distal end of a frame of an implant that is to be delivered to a treatment site, such as proximate to a valve annulus. Each anchor housing includes an anchor sleeve disposed within a bore of the anchor housing. The anchor sleeve includes a lumen extending therethrough including features disposed on an internal wall of the lumen for translatably supporting at least one anchor. The anchor may include a proximal anchor head that is releasably couplable to an anchor drive tube, where actuation (such as rotation or translation) of the anchor drive tube on the anchor results in translation of the anchor through the anchor sleeve towards a treatment site.

The anchor sleeve includes a collar disposed on its proximal end and configured to limit the extent of distal travel of the anchor within the sleeve. For example, a diameter of the lumen of the anchor sleeve that is surrounded by the collar may be less than a diameter of the proximal anchor head such that when the anchor is distally translated through the sleeve, further distal travel is impeded by the interaction of the proximal anchor head and the collar of the anchor sleeve. Thus, the collar of the anchor sleeve is used to limit the distal translation of the anchor through the anchor housing to adapt the anchor depth to the patient anatomy.

Further distal translation of the anchor through the anchor housing may be achieved by translating the anchor sleeve through the anchor housing. The collar of the anchor sleeve includes features that enable releasable engagement of the collar with the anchor drive tube. Thus, once the progress of the proximal anchor head becomes impeded by the collar of the anchor sleeve, the drive tube acts upon the features disposed on the collar to rotate the anchor sleeve and anchor in unison to distally advance the anchor sleeve, and as a result the anchor, through the bore of the anchor housing.

The use of an anchor sleeve with the anchor housing provides adaptable control over the depth of delivery of an anchor, enabling a surgeon to customize anchor depth in accordance with the variabilities of the anatomy at the treatment location. For example, anchors that are to be delivered to valve tissue having minimal thickness may advance the anchor sleeve only partway through the anchor housing. Rather than leaving the unused anchor exposed, thereby increasing the thrombus and other embolic risks, the anchor sleeve encases the anchor threads, minimizing such risks and increasing the resiliency and strength of the implant to protect against fatigue. However, for treatment locations where the valve tissue is thicker, the disclosed anchor housing assembly permits the anchor sleeve and anchor to travel to their maximum distal extent, to utilize full anchoring capabilities.

These and other beneficial aspects of the disclosed anchor housing assembly are described in more detail below. Although embodiments of the present disclosure may be described with specific reference to mitral valves, an anchor housing such as that disclosed herein that provides customizable depth anchoring may be readily adapted to facilitate reconstruction of any valve annulus, for example including a tricuspid valve annulus and/or may similarly benefit any other dilatation, valve incompetency, valve leakage and other similar heart failure conditions which involve anchoring a component to heart tissue.

As used herein, the term "distal" refers to the end farthest away from the medical professional when introducing a medical device into a patient, while the term "proximal" refers to the end closest to the medical professional when introducing a medical device into a patient.

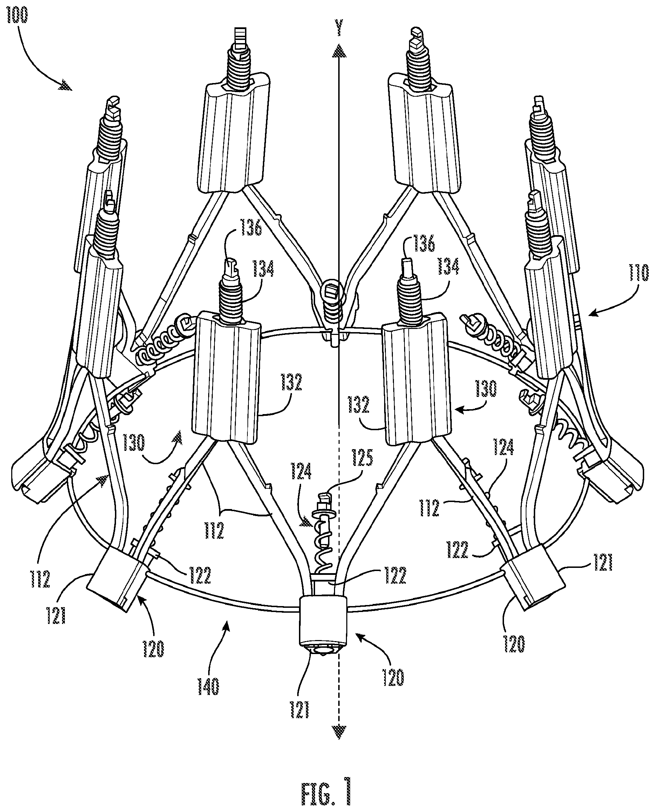

FIG. 1 illustrates an implant 100 comprising a frame 110 that may be disposed about a heart valve or other cardiac feature. For purposes of clarity not all of the components of the implant are numbered. In one embodiment, the frame 110 may extend circumferentially around and partially axially along a central frame axis extending proximally-distally through a center point of the frame. The frame 110 may be generally symmetric with respect to the central frame axis although it need not be symmetric. The frame 110 may form a generally tubular shape, where herein "tubular" includes circular as well as other rounded or otherwise closed shapes. The frame 110 may be configured to change shape, size and/or configuration. For example, the frame 110 may assume various shapes, sizes, configurations etc. during different phases of deployment such as during pre-delivery, delivery, tissue engagement and cinching.

According to one embodiment, the frame 110 may be formed from one or more struts 112 that may form all or part of the frame 110, where the struts 112 may include elongated structural members formed of a metal alloy, a shape memory material, such as an alloy of nickel titanium or other metals, metal alloys, plastics, polymers, composites, other suitable materials, or combinations thereof. In FIG. 1 sixteen struts 112 are shown although it is appreciated that in some embodiments, there may be fewer or more than sixteen struts.

In one embodiment, the struts112 of the frame 110 may be formed from the same, monolithic piece of material. Thus, reference to struts 112 may refer to different portions of the same, extensive component. Alternatively, reference to struts 112 may refer to components that are formed separately and attached permanently together, for example by welding or other methods. In some embodiments, the struts 112 may be separate components that are detachably coupled together to form proximal and distal apices. For example, the struts 112 are shown joined at their proximal apex by actuator 130 and at their distal apex by anchor housing assemblies 120.

In one embodiment, the actuator 130 includes an actuator shaft 134 that is rotatably carried by the proximal end of the frame 110, for example, a head of the actuator shaft 134 may be carried by a window or other opening (not shown) at the proximal apex of the frame 110. The actuator shaft 134 may include a drive coupler 136 at the proximal end. The actuator 130 may further include an actuator collar 132 having internal features configured to interact with the features of the actuator shaft 134 such that rotation of the actuator shaft 134 by an actuator drive tube coupled to the drive coupler 136 axially translates the actuator collar 132 over the actuator shaft 134 and over struts 112. In some embodiments, "axial" as applied to axial movement or restraint of the actuator collar includes directions that are at least partially in the proximal or distal direction and that are parallel or generally parallel to a central axis extending through (proximally - distally) the frame, such as at least partially in along axis Y. As shown in FIG. 1, struts 112 extend away from the proximal apex in opposing directions. Distal advancement of the actuator collar 132 over struts 112 pulls the struts together within the actuator collar 132, and as a result may be used to reduce the distance between anchor housing assemblies 120 to reshape heart tissue anatomy, for example to restore a valve to its native configuration. In one embodiment, each actuator collar 132 may be independently actuated in accordance with the reshaping objective for the associated anchor pair.

The implant further includes anchor housing assemblies 120. In the embodiment of FIG. 1, the anchor housing assemblies are shown coupled by a cinch cord 140. In some embodiments, the cinch cord 140 may be made of nylon or other suture material and may be used to retain and/or change relative positions of the anchor housing assemblies 120.

Each anchor housing assembly 120 is shown to include an anchor housing 121, an anchor sleeve 122 and an anchor 124. Each anchor 124 may include an anchor drive coupler 125 configured to connect the anchor 124 to an anchor drive tube. In accordance with the invention, the anchor 124 is translatably supported by the anchor sleeve 122 which in turn is translatably supported by the anchor housing 121. Following or prior to advancement of each actuator collar 132 over the struts 112, the anchors 124 may be driven through the anchor sleeve 122 and anchor housing 121 to embed distal tips of the anchors 124 into tissue.

According to one aspect, the anchor housing assembly 120 disclosed herein in various embodiments enables a surgeon to customize an extent of travel of the anchor 124 through the anchor housing 121 to thereby adapt anchoring depth in accordance with the variability of tissue anatomy.

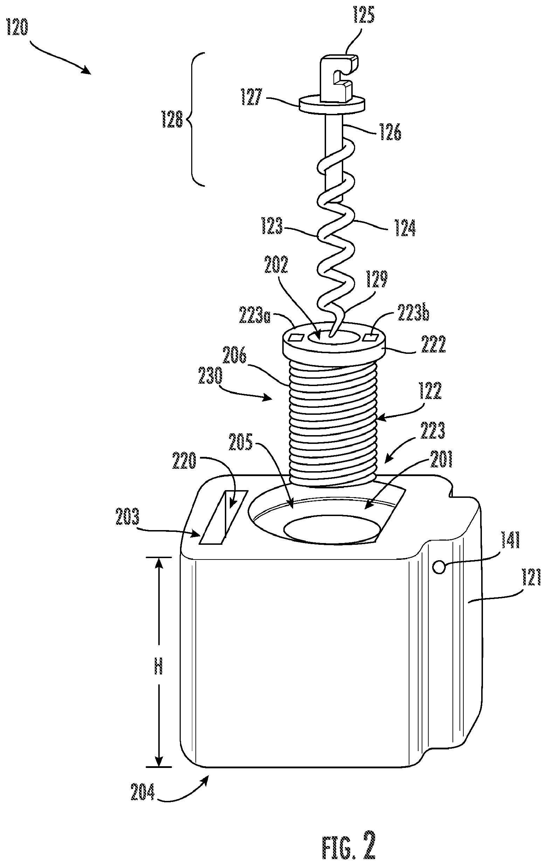

FIG. 2 illustrates one embodiment of an anchor housing assembly 120 in more detail. The anchor housing assembly 120 is shown to include an anchor housing 121 including a bore 201 extending therethrough. The anchor housing 121 may also include a frame sleeve 220 configured to accept one or more struts of a frame 110 (FIG. 1) to couple the anchor housing assembly 120 to the frame 110. In other embodiments, the anchor housing assembly may be integral with the frame 110. In some embodiments, the anchor housing assembly may include a cinch cord lumen 141 extending through a portion of the anchor housing for supporting the cinch cord 140 (FIG. 1) of the implant.

The bore 201 extends from a proximal surface 203 through a distal surface 204 of the anchor housing 121. The bore 201 includes features 205, such as ridges, threads or other types of features disposed on an internal wall of the bore 201. The features 205 disposed on the internal wall of the bore 201 may cooperate with features 206 disposed on an external surface of the anchor sleeve 122 to axially translate the anchor sleeve 122 through the bore 201 of the anchor housing 121.

The anchor sleeve 122 is comprised of a collar 222 disposed at its proximal end and a sleeve shaft 230 extending from the collar 222 to a distal end 233 of the anchor sleeve 122. The collar 222 has an opening extending therethrough defining a lumen 202 that extends from the collar 222 through the distal end 233 of the sleeve shaft 230. In some embodiments, the diameter of the collar 222 may be greater than the diameter of the bore 201 to limit distal translation of the anchor sleeve 122 past the proximal surface 203 of the anchor housing 121.

In accordance with the invention, the collar 222 includes one or more driver engagement features 223a, 223b disposed on a proximal surface of the collar 222. The driver engagement features 223a, 223b may include tabs, notches, ridges, flanges, hooks or other features that cooperate with complementary features of an anchor drive tube (not shown), enabling mating engagement of the collar 222 with the anchor drive tube and thus actuation of the anchor sleeve 122 by the anchor drive tube, for example to drive the anchor sleeve 122 through the bore 205 of the anchor housing 121.

An interior surface of the anchor sleeve 122 defining the lumen 202 has features disposed thereon to cooperate with an edge, ridge, thread or other feature of an anchor 124 to support axial translation of the anchor 124 through the anchor sleeve 122.

An anchor 124 is shown to include a proximal anchor head 128, a distal end 129 and a helical shaft 123 extending from the proximal anchor head 128 to the distal end 129. The proximal anchor head 128 includes a drive shaft 126 having a proximal anchor drive coupler 125 configured for mated engagement with the anchor drive tube (not shown). An anchor shaft collar 127 may extend radially outward from and at least partially around the drive shaft 126. **In** some embodiments, the diameter of the anchor shaft collar 127 may be greater than the diameter of the lumen 202 defined by anchor sleeve collar 222 to limit distal translation of the proximal anchor head 128 of the anchor 124 past the collar 222 of the anchor sleeve 122.

In some embodiments, the anchor 124, anchor sleeve 122 and anchor housing 121 may be made of a suitable biocompatible metal alloy such as stainless steel, cobalt chromium, platinum iridium, nickel titanium, other suitable materials, or combinations thereof. Each anchor 124 may be from about ten to about fifteen millimeters (mm) in total axial length. In some embodiments, the anchors 124 may be shorter or longer than ten to fifteen millimeters (mm) in total axial length. By "total" axial length it is meant the axial length of the anchor 124 from the end of the distal penetrating tip 129 to the opposite, proximal anchor head 128. The helical shaft 123 of the anchor 124 may be from about six to about twelve millimeters (mm) in axial length. In some embodiments, the helical shaft 123 may be shorter or longer than six to twelve millimeters (mm) in axial length. The proximal anchor head 128 and/or other non-helical portions of the anchor 124 may be from about three to about four millimeters (mm) in axial length. In some embodiments, the proximal anchor head 128 and/or other non-helical portions may be shorter or longer than three to four millimeters (mm) in axial length.

In some embodiments, the distal end 129 of the anchors 124 may be capable of extending from about four to about seven millimeters (mm) axially beyond the corresponding distal surface 204 of the anchor housing 121, enabling the helical shaft 123 to extend from between four to seven millimeters (mm) into the cardiac tissue.

In some embodiments, the anchor housing 121 may have a height H ranging from 2.5 mm to 5 mm. The shaft 230 of the anchor sleeve 122 may be matched to the height H such that it extends through the entire bore 201 of the anchor housing 121 although it is not required that the shaft 230 of the anchor sleeve be matched to the height H of the anchor housing 121. It is appreciated that the anchor sleeve 122 is used to adapt the depth of an associated anchor, for example to decrease the depth of anchor insertion for target treatment sites with fragile anatomy. Thus, in some embodiments, the anchor sleeve 122 may be used to decrease the depth of an anchor below the four millimeters (mm).

It should be noted that the disclosure is not limited to embodiments wherein the length of the shaft 230 of the anchor sleeve 121 is matched to the height H of the anchor housing 121 but may further include embodiments where the length of the anchor shaft 230 is between 0.25 - 1 times the height H of the anchor housing.

In one example of an embodiment, the height H of the anchor housing may be, for example 3 mm, the length of the anchor sleeve may be 3 mm, and the length of the anchor may be 7 mm from distal tip to collar 127, allowing anchors to be delivered having a range of anchor depths from between 1 and 4 mm.

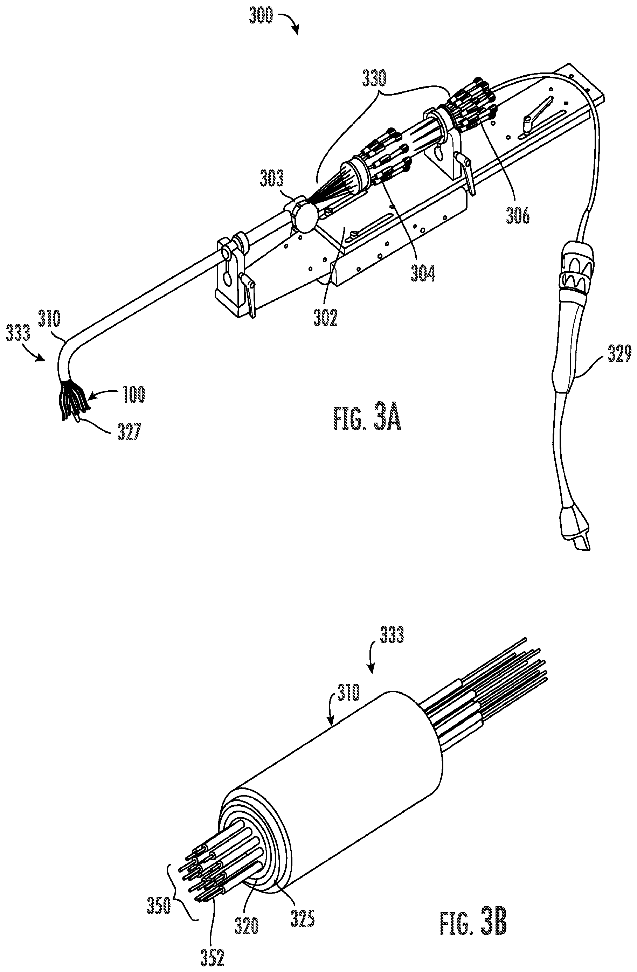

FIG. 3A illustrates an example of a deployment system 300 that may be used to deploy an implant 100 including anchor housings assemblies as described with regard to FIGS. 1 and 2.

In one embodiment, the deployment system may include an introducer sheath 310 comprised of a composite of layers of thermoplastic elastomer (TPE), for example PEBAX provided by ARKEMA corporation of Colombes France. Alternatively, nylon, polyurethanes, polyester, silicone or other similar materials may be used. In some embodiments, the length of the introducer shaft 310 is selected to enable a minimally invasive transluminal (such as transfemorally or transeptally) introduction of a distally carried implant into a cardiac cavity. Thus, the length of the introducer shaft 310 may range from between 24"-52", and more particularly between 42"-46". In one embodiment, the inner diameter may range between 24-31 Fr, and the outer diameter may range between 26 Fr and 34 Fr or more. In an example of an embodiment, an inner diameter may be, for example 28 Fr and the outer diameter may be 32 Fr.

In one embodiment, the introducer sheath 310 may be a steerable sheath enabling transluminal navigation to a cardiac treatment site. A proximal handle 330 may include a variety of controls to enable steering of the sheath 310 and operation of components coupled to the sheath 310. For example, the proximal handle may include a sheath steering knob 303, anchor knobs 304 and cinch knobs 306. The introducer sheath 310 may include steering wires that extend from the steering knob 303 of the proximal handle 330 to the distal end 333 of the introducer sheath 310, where rotation of the steering knob 303 may control deflection of the distal end 333 of the introducer sheath 310 during transluminal navigation.

The proximal handle 330 may be supported by a stage 302 and further include driver control mechanisms, such as cinch knobs 306 and anchor knobs 304. As described in more detail below, the introducer sheath may carry a plurality of drive tubes. Each drive tube may comprise a hypo tube or similar device configured to transmit a torque along its length to control implantation of the implant 100. For example, cinch knobs 306 may be used to control actuator drive tubes coupled to actuator shafts 134 to move actuators 130 over struts 112 (FIG. 1). Anchor knobs 304 may be used to control anchor drive tubes coupled to proximal anchor heads of anchors 124 (FIGS. 1 and 2), such that rotation of the anchor knobs 304 may rotationally advance the anchors 124 into the anchor housing assemblies 120. In addition, rotation of the anchor knobs 304 may cause anchor drive tubes to rotationally advance the anchor sleeves into the anchor housings as described in more detail below.

In some embodiments, an intravascular cardiac echography (ICE) catheter 327 may be disposed within the implant 100. The ICE catheter 327 may be coupled to an ICE catheter control handle 329 to monitor anchor placement during implant deployment.

Figure 3B illustrates one embodiment of a distal end 333 of introducer sheath 310 which carries a delivery catheter 320 within a working catheter 325, where the delivery catheter is shown to include channels supporting drive tubes 350. In one embodiment, the drive tubes 350 may include actuator drive tubes and anchor drive tubes. The drive tubes 350 may include drive couplers 352 for coupling the respective drive tube to the actuator, anchor, or other component of the deployment system 300.

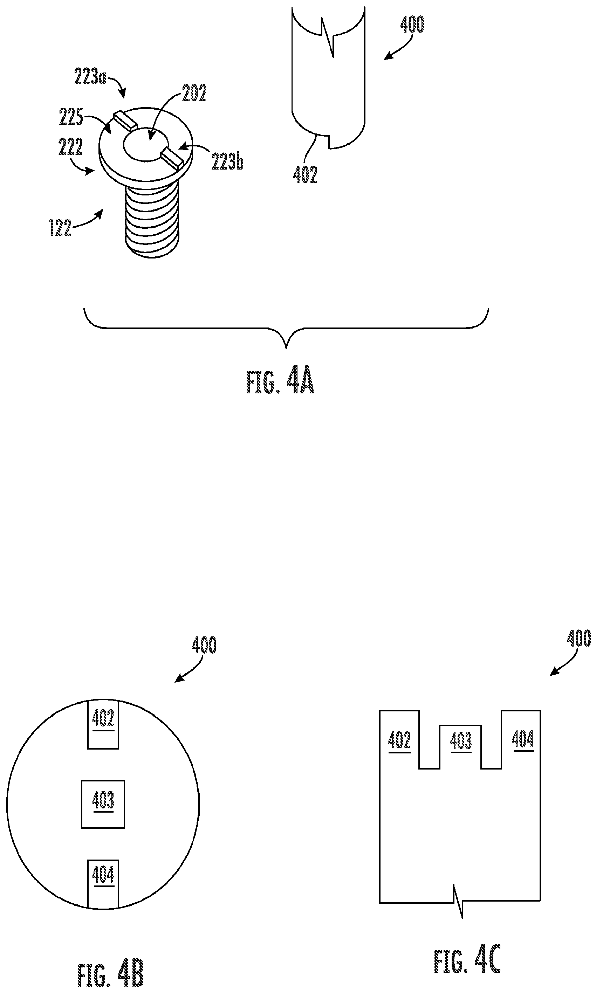

FIGS. 4A - 4C are perspective views of one embodiment of an anchor sleeve/ anchor drive tube coupling mechanism that may be used as disclosed herein. The anchor sleeve 122 is shown to include the anchor collar 222. A lumen 202 extends through the anchor sleeve 122. One or more drive tube engagement features 223a, 223b may be formed on our coupled to a proximally facing surface 225 of the anchor collar 222. Also shown in FIG. 4A is a distal end of an anchor drive tube 400, where a sleeve driver feature 402 is shown disposed at its distal end.

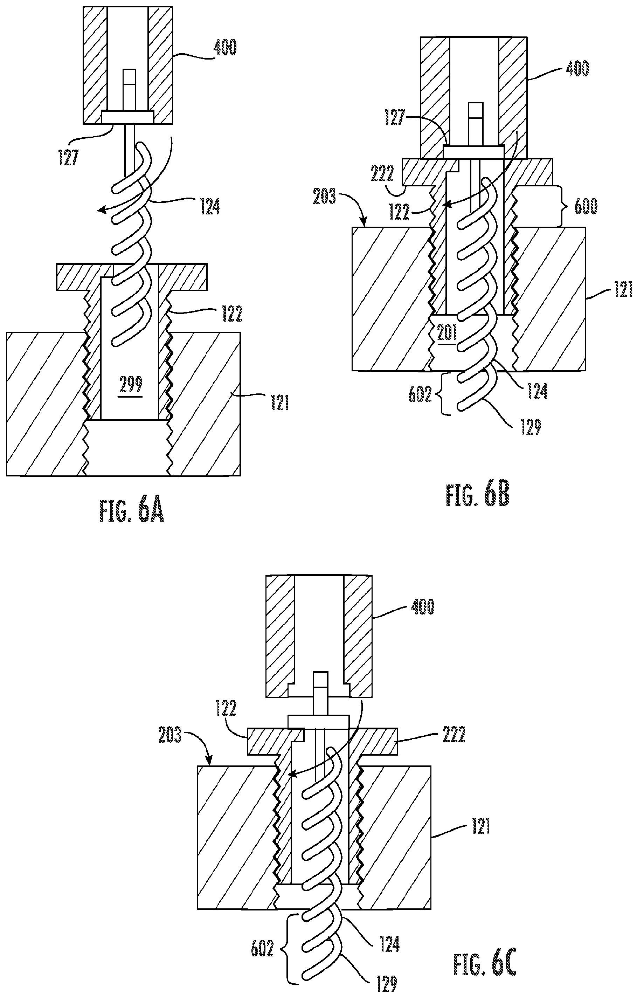

FIG. 4B is a proximally facing view of the distal end of the anchor drive tube 400, in which sleeve engagement feature 402 and sleeve engagement feature 404 are shown disposed about an outer circumference of the anchor drive tube. In one embodiment, an anchor drive coupler 403, configured to couple with the drive coupler 125 of the proximal anchor head 128 (FIG. 2) may be disposed in an inner circumference of the distal end of the anchor drive tube 400. FIG. 4C is a side perspective view of one embodiment of a distal end of an anchor driver, where the anchor drive coupler 403 is shown disposed between the sleeve engagement features 402, 404. As will be described in more detail with regards to FIGS. 6A-6C, when the anchor drive tube 400 drives the proximal head of the anchor through the lumen 202 of the sleeve 122, as the proximal head of the anchor approaches the collar 222, the sleeve driver features 402, 404 of the anchor driver 400 contact the anchor driver features 223a, 223b of the collar 222, enabling force from the drive tube to be transferred to the anchor sleeve 122 by the interaction of the anchor drive features 223a, 223b and the sleeve engagement features 402, 404. With such an arrangement, the anchor drive tube 400 may be used to drive both the anchor and the anchor sleeve.

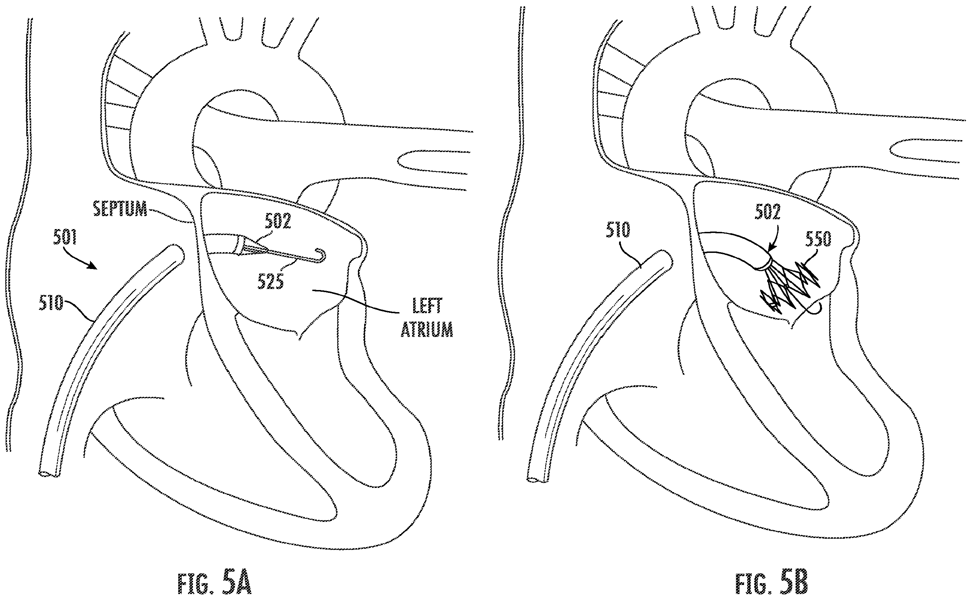

FIGS. 5A, 5B are sequential perspective views of one method of delivering an implant including adaptable anchor depth control as described herein, for example and implant for a valve annulus. The implant may be inserted using a delivery system such as that described with regard to FIG. 3A., to deliver an implant to a cardiac cavity, for example via access to the vasculature of the leg, such as the femoral vein or the iliac vein.

As shown in FIG. 5A, such a delivery system 501 may include a delivery catheter 510 comprising a guidewire 525 at its distal end that is used to navigate the distal end 502 of the delivery catheter 510 into the left atrium of a heart to deploy an implant for mitral valve repair. In FIG. 5B, an implant 550, for example an implant including anchor housings configured to adapt anchor depth as disclosed herein, may be released from the distal end 502 of the delivery catheter 510, for example by expelling the implant from the delivery catheter 510 or withdrawing the introducer sheath to expose the implant 100. The implant 550 may be positioned at a treatment site, such as a location proximate to, surrounding or partially surrounding a mitral valve annulus.

FIGS. 6A - 6C illustrate sequential views of one embodiment of a method of deploying anchors to a treatment site using the adaptable depth anchor housing assemblies disclosed herein, wherein the anchor housing 121, anchor sleeve 122 and anchor driver 400 are shown in cross section. In FIG. 6A, the anchor drive tube 400 actuates the anchor 124, for example by rotating the anchor. The anchor 124 interacts with edges, ridges, or other threads on an internal surface 299 of the anchor sleeve 122 to translate the anchor 124 through the anchor sleeve 122. The anchor drive tube 400 may continue to actuate the anchor 124 until, as shown in FIG. 6B, the anchor shaft collar 127 contacts the anchor sleeve collar 222, limiting further translation of the anchor 124 within the anchor sleeve 122. In one embodiment, the sleeve is initially disposed within the bore 201 of the anchor housing 121 such that the anchor collar 222 of the anchor sleeve 122 is spaced apart from the proximal surface 203 of the anchor housing 121 by a sleeve exposure extent 600. It is appreciated that the sleeve exposure extent is a matter of design choice that takes into consideration a length of an anchor and a minimum desired depth of the anchor in tissue. For example, as shown in FIG. 6B, when the anchor shaft collar 127 initially contacts the collar 222, the distal tip 129 of the anchor 124 extends distally from the anchor housing 121 by a distal extent 602. The anchor drive tube 400 may translate the anchor sleeve 121 proximally or distally to vary the distal extend 602 of the anchor exposed from the anchor housing 121. For example, FIG. 6C illustrates an anchor housing 121 where continued rotation of the anchor drive tube 400 has advanced the anchor sleeve 122 into the bore of the anchor housing 121, such that the distal extent 602 of the anchor is increased. The anchor drive tube 400 may continue to activate the anchor sleeve 122 until the anchor collar 222 contacts the proximal surface 203 of the anchor housing, limiting the extent of travel of the distal end 129 of the anchor 124 into tissue.

In one embodiment, the anchor drive tube may be configured to release the sleeve engagement features 402, 404 (FIGS. 4A-4C) of the anchor collar 222. For example, the sleeve engagement features 402, 404 may be retractable within the anchor drive tube 400. Retraction of the sleeve engagement features 402, 404 advantageously enables further rotation of the anchor 124 into tissue. Further rotation of the anchor 124 into tissue when distal translation of the anchor is limited by the interaction of the anchor collar 222 and the anchor shaft collar 127 may advantageously cinch tissue to improve anchor affixation.

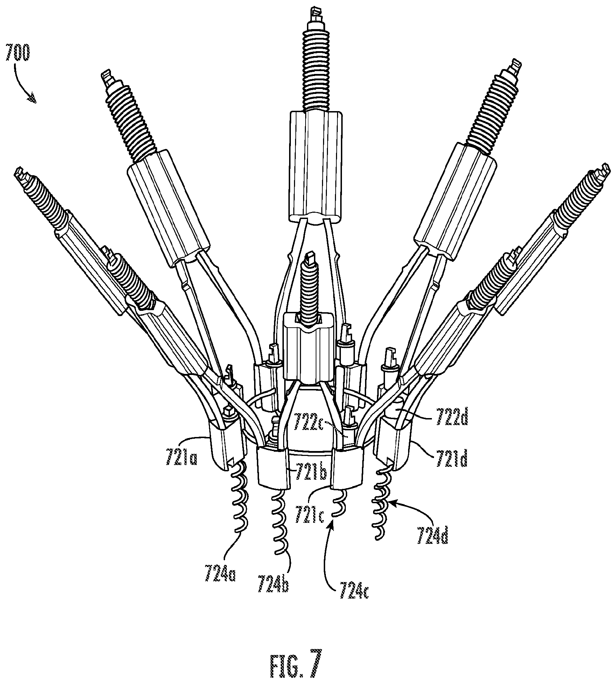

FIG. 7 illustrates one embodiment of an implant with adaptable anchor depth control mechanisms as disclosed herein. In FIG. 7, and implant 700 is shown to include a plurality of anchors 724a-724d, where the anchors 724a-724d have been advanced to different depths through their respective anchor housings 721a-721d. For example, anchors 724c and 724d have been advanced to a smaller distal extent than anchors 724a and 724b. As a result, the anchor sleeves 722c and 722d are exposed from anchor housings 721c and 721d, which reduce the exposure of the cardiac cavity to exposed threads of the anchors 724c and 724d.

Various modifications to the implementations described in this disclosure will be readily apparent to those skilled in the art, and the generic principles defined herein can be applied to other implementations without departing from the teaching of this disclosure. Thus, the disclosure is not intended to be limited to the implementations shown herein but is to be accorded the widest scope defined by the claims, the principles and the novel features disclosed herein. The word "example" is used exclusively herein to mean "serving as an example, instance, or illustration." Any implementation described herein as "example" is not necessarily to be construed as preferred or advantageous over other implementations, unless otherwise stated. Certain features that are described in this specification in the context of separate implementations also can be implemented in combination in a single implementation. Conversely, various features that are described in the context of a single implementation also can be implemented in multiple implementations separately or in any suitable sub-combination. Moreover, although features can be described above as acting in certain combinations and even initially claimed as such, one or more features from a claimed combination can in some cases be excised from the combination, and the claimed combination can be directed to a sub-combination or variation of a sub-combination.

Similarly, while operations are depicted in the drawings in a particular order, this should not be understood as requiring that such operations be performed in the particular order shown or in sequential order, or that all illustrated operations be performed, to achieve desirable results. In some cases, the actions recited in the claims can be performed in a different order and still achieve desirable results.

It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

## Claims

1. A coronary implant (100, 550, 700) comprising:
a frame (110) having a proximal end and a distal end;
an anchor (124, 724); and
an anchor housing (121, 721) coupled to one of the proximal end or distal end of the frame (110), the anchor housing (121, 721) including an anchor sleeve (122, 722) disposed within a bore (201) of the anchor housing (121, 721), the anchor sleeve (122, 722) having a lumen (202) extending therethrough including at least one feature disposed on an internal wall (299) of the lumen (202) for translatably engaging the anchor (124, 724);
the anchor sleeve (122, 722) translatable within the bore (201) of the anchor housing (121, 721) and configured to limit translation of the anchor (124, 724) through the anchor housing (121, 721),
wherein the anchor sleeve (122, 722) comprises a collar (222) disposed on a proximal end of the anchor sleeve (122, 722) to limit the translation of the anchor sleeve (122, 722) within the bore (201) or the translation of the anchor (124, 724) within the anchor sleeve (122, 722), or both, **characterised in that** the collar (222) comprises a proximal surface and at least one driver engagement feature (223a, 223b) disposed on the proximal surface.

2. The coronary implant of any of claims 1, wherein the anchor (121 721) further includes an anchor head (128) disposed at a proximal end, the anchor head (128) comprising a drive coupler (136).

3. The coronary implant of any of claims 1-2 as part of an implant deployment system (300, 501) including a delivery catheter (310, 510) comprising a proximal handle (330), a distal end (333, 502), and a drive tube (350, 400) extending from the proximal handle (330) to the distal end (333, 502), the drive tube (350, 400) comprising a distal driver (352) configured to releasably couple the anchor head (128) to drive the anchor (124, 724) through the lumen (202) of the anchor sleeve (122, 722).

4. The coronary implant of claim 3, wherein the distal driver (352) includes a sleeve engagement feature (402, 404) extending radially from its distal end, the sleeve engagement feature (402, 404) configured to cooperate with the driver engagement feature (223a, 223b) on the proximal surface of the collar (222) to translate the anchor sleeve (122, 722) within the bore (201) of the anchor housing (121, 721).

5. The coronary implant of any of claims 1-4, wherein the anchor head (128) comprises a distal anchor head diameter that is larger in size than a lumen diameter of the lumen of the anchor sleeve (122, 722) to preclude distal translation of the anchor head (128) into the lumen of the anchor sleeve (122, 722).

6. The coronary implant of any of claims 1-4, wherein the collar (222) extends radially from the lumen of the anchor sleeve (122, 722) and a collar diameter of the collar is larger than a bore diameter of the bore (201) of the anchor housing (121, 721) to limit distal translation of the anchor sleeve (122, 722) into the bore (201).

7. The coronary implant of any of claims 1-4, wherein the anchor sleeve (121, 721) comprises at least one external engagement feature disposed on an external surface of the anchor sleeve (121, 721), the bore (201) comprises at least one internal engagement feature disposed on a wall of the bore, and the at least one external engagement feature of the anchor sleeve (122, 722) cooperates with the at least one internal engagement feature of the bore to translate the anchor sleeve (122, 722) within the bore of the anchor housing (121, 721).

8. The coronary implant of claim 7, wherein the at least one feature of the lumen of the anchor sleeve (122, 722) cooperates with at least one edge of the anchor (124, 724) to translate the anchor (124, 724) within the anchor sleeve (122, 722).

9. The coronary implant of any of claims 1-4, wherein the frame (110) comprises an expandable frame comprising a plurality of struts (112) joined at distal ends to form distal apices, the anchor housing (121, 721) is one of a plurality of anchor housings (121, 721) that are disposed at the distal apices of the frame and the anchor (124, 724) is one of a plurality of anchors (124, 724), each anchor (124, 724) disposed within one of the plurality of anchor housings (121, 721).

10. The coronary implant of claim 9 wherein at least two of the plurality of struts of the frame (110) are joined at proximal ends to provide a proximal apex, and wherein the implant comprises an actuator (130) translatably disposed about the proximal apex to compress the frame (110).

11. The coronary implant of claim 10 as part of an implant deployment system (300, 501) including a delivery catheter (310, 510) comprising a proximal handle (330), a distal end (333, 502), and a plurality of drive tubes (350, 400) extending from the proximal handle (330) to the distal end (333, 502) of the delivery catheter, the plurality of drive tubes (350, 400) coupled to the plurality of anchors (124, 724), wherein the drive tubes (350, 400) are independently controllable at the proximal handle (330) to enable translation of anchor sleeves (122, 722) to different extents within the plurality of anchor housings (121, 721).

12. The coronary implant of claim 11, wherein each drive tube (350, 400) includes a first release mechanism for releasing the drive tube from the anchor sleeve (122, 722) and a second release mechanism for releasing the drive tube (350, 400) from the anchor (124, 724), in particular wherein the anchor sleeve (122, 722) is configured to preclude translation of the anchor (124, 724) while enabling rotation of the anchor (124, 724).

13. The coronary implant of any of claims 2-12, wherein the anchor head (128) comprises a distal surface having a sleeve engagement feature (402, 404) disposed thereon, the sleeve engagement feature (402, 404) configured to cooperate with a driver engagement feature (223a, 223b) of a drive tube (350, 400) to translate the anchor sleeve (122, 722) within the bore of the anchor housing (121, 721).

14. The coronary implant of any of claims 1-13, wherein the anchor housing (121, 721) is one of a plurality of anchor housings (121, 721), the anchor sleeve (122, 722) is one of a plurality of anchor sleeves (122, 722), the anchor (124, 724) is one of a plurality of anchors (124, 724), and wherein the plurality of anchor housings (121, 721) are disposed about the frame (110) and each anchor housing (121, 721) engages one of the plurality of anchor sleeves (122, 722) which supports one of the plurality of anchors (124, 724), and wherein each of the plurality of anchors (124, 724) are coupled to one drive tube (350, 400) of a plurality of drive tubes (350, 400) for independent translation of the coupled anchor (124, 724) and an associated anchor sleeve (122, 722).

15. The coronary implant of any of claims 3-14, wherein each drive tube (350, 400) of the plurality of drive tubes (350, 400) is independently controlled to customize translation of the anchor (124, 724), translation of the anchor sleeve (122, 722), or both.

## Patentansprüche

1. Koronarimplantat (100, 550, 700), umfassend:
einen Rahmen (110) mit einem proximalen Ende und einem distalen Ende,
einen Anker (124, 724) und
ein Ankergehäuse (121, 721), das an das proximale Ende oder das distale Ende des Rahmens (110) gekoppelt ist, wobei das Ankergehäuse (121, 721) eine Ankerhülse (122, 722) aufweist, die in einer Bohrung (201) des Ankergehäuses (121, 721) angeordnet ist, wobei die Ankerhülse (122, 722) ein Lumen (202) aufweist, das sich dort hindurch erstreckt und mindestens ein Merkmal aufweist, das an einer Innenwand (299) des Lumens (202) zur translatorischen Ineingriffnahme des Ankers (124, 724) angeordnet ist,
wobei die Ankerhülse (122, 722) in der Bohrung (201) des Ankergehäuses (121, 721) translatierbar und zur Begrenzung der Translation des Ankers (124, 724) durch das Ankergehäuse (121, 721) ausgestaltet ist,
wobei die Ankerhülse (122, 722) einen Bund (222) umfasst, der an einem proximalen Ende der Ankerhülse (122, 722) angeordnet ist, um die Translation der Ankerhülse (122, 722) in der Bohrung (201) oder die Translation des Ankers (124, 724) in der Ankerhülse (122, 722) oder beides zu begrenzen, **dadurch gekennzeichnet, dass** der Bund (222) eine proximale Fläche und mindestens ein Mitnehmereingriffsmerkmal (223a, 223b) umfasst, das an der proximalen Fläche angeordnet ist.

2. Koronarimplantat nach Anspruch 1, wobei der Anker (121, 721) ferner einen Ankerkopf (128) aufweist, der an einem proximalen Ende angeordnet ist, wobei der Ankerkopf (128) einen Antriebskoppler (136) umfasst.

3. Koronarimplantat nach einem der Ansprüche 1 - 2 als Teil eines Implantatablagesystems (300, 501), das einen Zuführkatheter (310, 510) aufweist, der einen proximalen Griff (330), ein distales Ende (333, 502) und ein Antriebsrohr (350, 400), das sich von dem proximalen Griff (330) zu dem distalen Ende (333, 502) erstreckt, umfasst, wobei das Antriebsrohr (350, 400) einen distalen Mitnehmer (352) umfasst, der dazu ausgestaltet ist, den Ankerkopf (128) lösbar zu koppeln, um den Anker (124, 724) durch das Lumen (202) der Ankerhülse (122, 722) zu treiben.

4. Koronarimplantat nach Anspruch 3, wobei der distale Mitnehmer (352) ein Hülseneingriffsmerkmal (402, 404) aufweist, das sich radial von seinem distalen Ende erstreckt, wobei das Hülseneingriffsmerkmal (402, 404) dazu ausgestaltet ist, mit dem Mitnehmereingriffsmerkmal (223a, 223b) an der proximalen Fläche des Bunds (222) zusammenzuwirken, um die Ankerhülse (122, 722) in der Bohrung (201) des Ankergehäuses (121, 721) zu translatieren.

5. Koronarimplantat nach einem der Ansprüche 1 - 4, wobei der Ankerkopf (128) einen distalen Ankerkopfdurchmesser umfasst, der größer als ein Lumendurchmesser des Lumens der Ankerhülse (122, 722) ist, um eine distale Translation des Ankerkopfs (128) in das Lumen der Ankerhülse (122, 722) auszuschließen.

6. Koronarimplantat nach einem der Ansprüche 1 - 4, wobei sich der Bund (222) radial von dem Lumen der Ankerhülse (122, 722) erstreckt und ein Bunddurchmesser des Bunds größer als ein Bohrungsdurchmesser der Bohrung (201) des Ankergehäuses (121, 721) ist, um eine distale Translation der Ankerhülse (122, 722) in die Bohrung (201) zu begrenzen.

7. Koronarimplantat nach einem der Ansprüche 1 - 4, wobei die Ankerhülse (121, 721) mindestens ein äußeres Eingriffsmerkmal umfasst, das an einer Außenfläche der Ankerhülse (121, 721) angeordnet ist, die Bohrung (201) mindestens ein inneres Eingriffsmerkmal umfasst, das an einer Wand der Bohrung angeordnet ist, und das mindestens eine äußere Eingriffsmerkmal der Ankerhülse (122, 722) mit dem mindestens einen inneren Eingriffsmerkmal der Bohrung zusammenwirkt, um die Ankerhülse (122, 722) in der Bohrung des Ankergehäuses (121, 721) zu translatieren.

8. Koronarimplantat nach Anspruch 7, wobei das mindestens eine Merkmal des Lumens der Ankerhülse (122, 722) mit mindestens einem Rand des Ankers (124, 724) zusammenwirkt, um den Anker (124, 724) in der Ankerhülse (122, 722) zu translatieren.

9. Koronarimplantat nach einem der Ansprüche 1 - 4, wobei der Rahmen (110) einen expandierbaren Rahmen umfasst, der eine Vielzahl von Streben (112) umfasst, die an distalen Enden verbunden sind, um distale Scheitelpunkte zu bilden, wobei das Ankergehäuse (121, 721) eines einer Vielzahl von Ankergehäusen (121, 721) ist, die an den distalen Scheitelpunkten des Rahmens angeordnet sind, und der Anker (124, 724) einer einer Vielzahl von Ankern (124, 724) ist, wobei jeder Anker (124, 724) in einem der Vielzahl von Ankergehäusen (121, 721) angeordnet ist.

10. Koronarimplantat nach Anspruch 9, wobei mindestens zwei der Vielzahl von Streben des Rahmens (110) an proximalen Enden verbunden sind, um einen proximalen Scheitelpunkt bereitzustellen, und wobei das Implantat einen Aktuator (130) umfasst, der translatorisch um den proximalen Scheitelpunkt angeordnet ist, um den Rahmen (110) zusammenzudrücken.

11. Koronarimplantat nach Anspruch 10 als Teil eines Implantatablagesystems (300, 501), das einen Zuführkatheter (310, 510) aufweist, der einen proximalen Griff (330), ein distales Ende (333, 502), und eine Vielzahl von Antriebsrohren (350, 400), die sich von dem proximalen Griff (330) zu dem distalen Ende (333, 502) des Zuführkatheters erstrecken, umfasst, wobei die Vielzahl von Antriebsrohren (350, 400) an die Vielzahl von Ankern (124, 724) gekoppelt sind, wobei die Antriebsrohre (350, 400) unabhängig an dem proximalen Griff (330) steuerbar sind, um eine Translation der Ankerhülsen (122, 722) in unterschiedlichen Ausmaßen in der Vielzahl von Ankergehäusen (121, 721) zu ermöglichen.

12. Koronarimplantat nach Anspruch 11, wobei jedes Antriebsrohr (350, 400) einen ersten Freigabemechanismus zum Lösen des Antriebsrohrs von der Ankerhülse (122, 722) und einen zweiten Freigabemechanismus zum Lösen des Antriebsrohrs (350, 400) von dem Anker (124, 724) aufweist, insbesondere wobei die Ankerhülse (122, 722) dazu ausgestaltet ist, eine Translation des Ankers (124, 724) zu verhindern, während eine Drehung des Ankers (124, 724) ermöglicht wird.

13. Koronarimplantat nach einem der Ansprüche 2 - 12, wobei der Ankerkopf (128) eine distale Fläche mit einem darauf angeordneten Hülseneingriffsmerkmal (402, 404) umfasst, wobei das Hülseneingriffsmerkmal (402, 404) dazu ausgestaltet ist, mit einem Mitnehmereingriffsmerkmal (223a, 223b) eines Antriebsrohrs (350, 400) zusammenzuwirken, um die Ankerhülse (122, 722) in der Bohrung des Ankergehäuses (121, 721) zu translatieren.

14. Koronarimplantat nach einem der Ansprüche 1 - 13, wobei das Ankergehäuse (121, 721) eines einer Vielzahl von Ankergehäusen (121, 721) ist, die Ankerhülse (122, 722) eine einer Vielzahl von Ankerhülsen (122, 722) ist, der Anker (124, 724) einer einer Vielzahl von Ankern (124, 724) ist, und wobei die Vielzahl von Ankergehäusen (121, 721) um den Rahmen (110) herum angeordnet sind und jedes Ankergehäuse (121, 721) eine der Vielzahl von Ankerhülsen (122, 722) in Eingriff nimmt, die einen der Vielzahl von Ankern (124, 724) stützt, und wobei jeder der Vielzahl von Ankern (124, 724) an ein Antriebsrohr (350, 400) einer Vielzahl von Antriebsrohren (350, 400) zur unabhängigen Translation des gekoppelten Ankers (124, 724) und einer zugeordneten Ankerhülse (122, 722) gekoppelt ist.

15. Koronarimplantat nach einem der Ansprüche 3 - 14, wobei jedes Antriebsrohr (350, 400) der Vielzahl von Antriebsrohren (350, 400) unabhängig gesteuert ist, um die Translation des Ankers (124, 724), die Translation der Ankerhülse (122, 722) oder beides individuell einzurichten.

## Revendications

1. Implant coronaire (100, 550, 700) comprenant :
un cadre (110), ayant une extrémité proximale et une extrémité distale ;
un ancrage (124, 724) ; et
un logement d'ancrage (121, 721) couplé à l'une de l'extrémité proximale ou de l'extrémité distale du cadre (110), le logement d'ancrage (121, 721) comprenant un manchon d'ancrage (122, 722) disposé dans un alésage (201) du logement d'ancrage (121, 721), le manchon d'ancrage (122, 722) ayant une lumière (202) s'étendant à travers celui-ci comprenant au moins un élément disposé sur une paroi interne (299) de la lumière (202) pour s'engager en translation avec l'ancrage (124, 724) ;
le manchon d'ancrage (122, 722) pouvant être déplacé en translation à l'intérieur de l'alésage (201) du logement d'ancrage (121, 721) et étant configuré pour limiter la translation de l'ancrage (124, 724) à travers le logement d'ancrage (121, 721),
le manchon d'ancrage (122, 722) comprenant un collier (222) disposé sur une extrémité proximale du manchon d'ancrage (122, 722) pour limiter le déplacement en translation du manchon d'ancrage (122, 722) à l'intérieur de l'alésage (201) ou le déplacement en translation de l'ancrage (124, 724) à l'intérieur du manchon d'ancrage (122, 722), ou les deux, **caractérisé en ce que** le collier (222) comprend une surface proximale et au moins un élément d'engagement de dispositif d'entraînement (223a, 223b) disposé sur la surface proximale.

2. Implant coronaire selon la revendication 1, l'ancrage (121, 721) comprenant en outre une tête d'ancrage (128) disposée à une extrémité proximale, la tête d'ancrage (128) comprenant un coupleur d'entraînement (136).

3. Implant coronaire selon l'une quelconque des revendications 1 et 2, en tant que partie d'un système de déploiement d'implant (300, 501) comprenant un cathéter de mise en place (310, 510) comprenant une poignée proximale (330), une extrémité distale (333, 502), et un tube d'entraînement (350, 400) s'étendant de la poignée proximale (330) à l'extrémité distale (333, 502), le tube d'entraînement (350, 400) comprenant un dispositif d'entraînement distal (352) configuré pour coupler de manière amovible la tête d'ancrage (128) pour entraîner l'ancrage (124, 724) à travers la lumière (202) du manchon d'ancrage (122, 722).

4. Implant coronaire selon la revendication 3, le dispositif d'entraînement distal (352) comprenant un élément d'engagement de manchon (402, 404) s'étendant radialement depuis son extrémité distale, l'élément d'engagement de manchon (402, 404) étant configuré pour coopérer avec l'élément d'engagement de dispositif d'entraînement (223a, 223b) sur la surface proximale du collier (222) pour déplacer en translation le manchon d'ancrage (122, 722) à l'intérieur de l'alésage (201) du logement d'ancrage (121, 721).

5. Implant coronaire selon l'une quelconque des revendications 1 à 4, la tête d'ancrage (128) comprenant un diamètre de tête d'ancrage distal qui est plus grand en taille qu'un diamètre de lumière de la lumière du manchon d'ancrage (122, 722) pour empêcher la translation distale de la tête d'ancrage (128) dans la lumière du manchon d'ancrage (122, 722).

6. Implant coronaire selon l'une quelconque des revendications 1 à 4, le collier (222) s'étendant radialement depuis la lumière du manchon d'ancrage (122, 722) et un diamètre de collier du collier étant plus grand qu'un diamètre d'alésage de l'alésage (201) du logement d'ancrage (121, 721) pour limiter la translation distale du manchon d'ancrage (122, 722) dans l'alésage (201).

7. Implant coronaire selon l'une quelconque des revendications 1 à 4, le manchon d'ancrage (121, 721) comprenant au moins un élément d'engagement externe disposé sur une surface externe du manchon d'ancrage (121, 721), l'alésage (201) comprenant au moins un élément d'engagement interne disposé sur une paroi de l'alésage, et l'au moins un élément d'engagement externe du manchon d'ancrage (122, 722) coopérant avec l'au moins un élément d'engagement interne de l'alésage pour déplacer en translation le manchon d'ancrage (122, 722) à l'intérieur de l'alésage du logement d'ancrage (121, 721).

8. Implant coronaire selon la revendication 7, l'au moins un élément de la lumière du manchon d'ancrage (122, 722) coopérant avec au moins un bord de l'ancrage (124, 724) pour déplacer en translation l'ancrage (124, 724) à l'intérieur du manchon d'ancrage (122, 722).

9. Implant coronaire selon l'une quelconque des revendications 1 à 4, le cadre (110) comprenant un cadre extensible comprenant une pluralité d'entretoises (112) jointes à des extrémités distales pour former des sommets distaux, le logement d'ancrage (121, 721) étant l'un d'une pluralité de logements d'ancrage (121, 721) qui sont disposés aux sommets distaux du cadre et l'ancrage (124, 724) étant l'un d'une pluralité d'ancrages (124, 724), chaque ancrage (124, 724) étant disposé à l'intérieur de l'un de la pluralité de logements d'ancrage (121, 721).

10. Implant coronaire selon la revendication 9, au moins deux de la pluralité d'entretoises du cadre (110) étant jointes à des extrémités proximales pour fournir un sommet proximal, et l'implant comprenant un actionneur (130) disposé en translation autour du sommet proximal pour comprimer le cadre (110).

11. Implant coronaire selon la revendication 10, en tant que partie d'un système de déploiement d'implant (300, 501) comprenant un cathéter de mise en place (310, 510) comprenant une poignée proximale (330), une extrémité distale (333, 502), et une pluralité de tubes d'entraînement (350, 400) s'étendant de la poignée proximale (330) à l'extrémité distale (333, 502) du cathéter de mise en place, la pluralité de tubes d'entraînement (350, 400) étant couplée à la pluralité d'ancrages (124, 724), les tubes d'entraînement (350, 400) pouvant être commandés indépendamment au niveau de la poignée proximale (330) pour permettre la translation de manchons d'ancrage (122, 722) sur différentes étendues à l'intérieur de la pluralité de logements d'ancrage (121, 721).

12. Implant coronaire selon la revendication 11, chaque tube d'entraînement (350, 400) comprenant un premier mécanisme de libération pour libérer le tube d'entraînement du manchon d'ancrage (122, 722) et un second mécanisme de libération pour libérer le tube d'entraînement (350, 400) de l'ancrage (124, 724), le manchon d'ancrage (122, 722) étant en particulier configuré pour empêcher la translation de l'ancrage (124, 724) tout en permettant la rotation de l'ancrage (124, 724).

13. Implant coronaire selon l'une quelconque des revendications 2 à 12, la tête d'ancrage (128) comprenant une surface distale ayant un élément d'engagement de manchon (402, 404) disposé sur celle-ci, l'élément d'engagement de manchon (402, 404) étant configuré pour coopérer avec un élément d'engagement de dispositif d'entraînement (223a, 223b) d'un tube d'entraînement (350, 400) pour déplacer en translation le manchon d'ancrage (122, 722) à l'intérieur de l'alésage du logement d'ancrage (121, 721).

14. Implant coronaire selon l'une quelconque des revendications 1 à 13, le logement d'ancrage (121, 721) étant l'un d'une pluralité de logements d'ancrage (121, 721), le manchon d'ancrage (122, 722) étant l'un d'une pluralité de manchons d'ancrage (122, 722), l'ancrage (124, 724) étant l'un d'une pluralité d'ancrages (124, 724), et la pluralité de logements d'ancrage (121, 721) étant disposés autour du cadre (110) et chaque logement d'ancrage (121, 721) s'engageant avec l'un de la pluralité de manchons d'ancrage (122, 722) qui supporte l'un de la pluralité d'ancrages (124, 724), et chacun de la pluralité d'ancrages (124, 724) étant couplé à un tube d'entraînement (350, 400) d'une pluralité de tubes d'entraînement (350, 400) pour une translation indépendante de l'ancrage couplé (124, 724) et d'un manchon d'ancrage associé (122, 722).

15. Implant coronaire selon l'une quelconque des revendications 3 à 14, chaque tube d'entraînement (350, 400) de la pluralité de tubes d'entraînement (350, 400) étant commandé indépendamment pour personnaliser le déplacement en translation de l'ancrage (124, 724), la translation du manchon d'ancrage (122, 722), ou les deux.
